# EUROPÄISCHE PATENTSCHRIFT

(11) **EP 1 255 578 B1**
(45) Veröffentlichungstag und Bekanntmachung des Hinweises auf die Patenterteilung: **31.05.2006**
(21) Anmeldenummer: 01951124.5
(22) Anmeldetag: 20.01.2001
(51) Int. Cl.: A61M 5/172, A61B 5/00

(54) **ANORDNUNG ZUR DOSIERUNG EINES DIE BLUTGLUKOSE EINES PATIENTEN REGULIERENDEN HORMONS**
ARRANGEMENT FOR DOSING A HORMONE REGULATING BLOOD SUGAR IN A PATIENT
SYSTEME POUR LE DOSAGE D'UNE HORMONE REGULANT LA GLYCEMIE D'UN PATIENT

(30) Priorität: 10.02.2000 DE 10006044
(43) Veröffentlichungstag der Anmeldung: 13.11.2002
(73) Patentinhaber: F.HOFFMANN-LA ROCHE AG, 4070 Basel (CH); Roche Diagnostics GmbH, 68305 Mannheim (DE)
(72) Erfinder: WITTMANN, Uwe, 68623 Lampertheim (DE); RINNE, Helmut, 68199 Mannheim (DE); GESSLER, Ralf, 88255 Baienfurt (DE); PFLEIDERER, Hans-Joerg, 89075 Ulm (DE)
(74) Vertreter: Pfiz, Thomas
(86) Internationale Anmeldenummer: PCT/EP2001/000625
(87) Internationale Veröffentlichungsnummer: WO 2001/058511

(56) Entgegenhaltungen:
- EP-A- 1 185 321
- DE-A- 19 756 872
- US-A- 4 280 494

## Beschreibung

Die Erfindung betrifft eine Anordnung zur Dosierung eines zur Regulierung der Blutglukose eines Patienten geeigneten Hormons, insbesondere Insulin.

Diabetes mellitus ist eine Stoffwechselerkrankung, bei welcher die Regulation des Glukosegehalts des Bluts auf einen den intermediären Stoffwechselbedürfnissen entsprechenden Pegel durch einen Mangel an Insulin gestört ist. Konventionell wirken Diabetespatienten einer Glukosekonzentrationserhöhung durch Injektion von körperfremdem Insulin entgegen. Die zu verabreichende Menge wird auf empirischer Basis anhand von Blutzuckerselbstkontrollen bestimmt. Es wurde bereits vorgeschlagen, die Zuckerbestimmung dadurch zu verbessern, daß im Fettgewebe mittels einer Mikrodialysesonde kontinuierlich oder intervallweise Proben entnommen und automatisch auf den Gewebeglukosespiegel als Maß für die Blutglukose ausgewertet werden.

Die DD 282 616 A5 beschreibt eine Einrichtung zur belastungsangepaßten Steuerung von Infusionspumpen, deren Insulinzufuhr zum Diabetiker entweder automatisch durch einen Blutglukosesensor oder halbautomatisch über patientenspezifische Insulindosierungsprofile mittels Mikrorechner gesteuert wird. Dabei ist an den Mikrorechner eingangsseitig ein Herzfrequenzsensor angekoppelt, um bei Überschreiten eines Grenzwertes eine Umsteuerung zwischen so genannter positiver und negativer Insulin-Glukose-Charakteristik zu ermöglichen. Damit soll während einer Belastungssituation anstelle der sensorischen Steuerung eine nach einer empirischen Funktion ermittelte zeitabhängige Verringerung der Dosierung im Sinne einer Senkung der Plasmainsulinkonzentration erfolgen.

Aus der EP-A 0 824 240 ist es bekannt, aus Belastungsherzfrequenzdaten auf der Grundlage eines physiologischen Modells des Glukose-/Insulin-Stoffwechsels rechnergestützt sogenannte individualspezifische Insulinwirk- . äquivalente zu bestimmen, welche für Lern- und Trainingsprogramme sowie in der Fort- und Weiterbildung einsetzbar sein sollen.

Die EP-A-1 185 321 fällt unter die Bestimmungen von Art. 54 (3) EPÜ. Es wird ein Regelkreis mit manneller Vorstenerung offenbart.

Ausgehend hiervon liegt der Erfindung die Aufgabe zugrunde, eine Anordnung und ein Verfahren der eingangs angegebenen Gattung anzugeben, womit die Dosierung eines Blutglukose beeinflussenden Hormons verbessert und unter Berücksichtigung des Patientenzustandes optimierbar ist. Insbesondere soll es dem Patienten damit ermöglicht werden, durch entsprechende Hormongaben seinen Blutzuckerspiegel dauerhaft im normoglykämischen Bereich zu halten.

Zur Lösung dieser Aufgabe wird die im Patentanspruch 1 angegebene Merkmalskombination vorgeschlagen. Vorteilhafte Ausgestaltungen und Weiterbildungen der Erfindung ergeben sich aus den abhängigen Ansprüchen.

Der Kern der Erfindung liegt darin, eine Regeleinrichtung zur Feinausregelung der Hormondosierung vorzusehen, während von der Regelung nur mit Zeitverschiebung erfaßbare Einflüsse durch antizipierende Grobvorsteuerung zumindest teilweise ausgeglichen werden. Dementsprechend wird erfindungsgemäß ein System mit folgenden Merkmalen vorgeschlagen:
- einer Meßeinrichtung zur Erfassung von mit der Blutglukose korrelierbaren Meßwerten;
- einer Regeleinrichtung, die einen Regler zur Verarbeitung der Meßwerte nach einem Regelalgorithmus sowie eine Hormondosiereinheit zur Ausgabe einer Hormondosis umfaßt;
- einer auf die Regeleinrichtung (12) einwirkenden Vorsteuereinrichtung (14) zur Grobvorsteuerung und Totzeitverminderung der Regelung nach Maßgabe einer den Blutglukosespiegel des Patienten beeinflussenden Einflußgröße.

Durch die Grobvorsteuerung kann die Regelgüte erheblich verbessert werden. Insbesondere können Änderungen der Stoffwechsellage aufgrund äußerer Einwirkungen oder im Körperinnern ablaufender Transportvorgänge quasi vorausschauend ohne Totzeit berücksichtigt werden, so daß Regelabweichungen auf einen kleinen Bereich begrenzt bleiben und überhöhte Blutglukosewerte a priori vermieden werden. Damit läßt sich eine optimale Dosierung zur normoglykämischen Einstellung der Blutglukose erreichen.

Dies kann vorteilhafterweise dadurch erfolgen, daß die Vorsteuereinrichtung nach Maßgabe einer sensorisch erfaßten Einflußgröße vorsteuernd auf die Regeleinrichtung einwirkt.

Gemäß einer besonders bevorzugten Ausgestaltung der Erfindung soll das Ausmaß einer Körpertätigkeit des Diabetikers dadurch Berücksichtigung finden, daß die Vorsteuereinrichtung eine Aktivitätsmeßeinheit zur sensorischen Erfassung des körperlichen Aktivitätsgrades des Patienten aufweist. Hinsichtlich einer differenzierten Erfassung von Ruhezuständen des Patienten wie Liegen, Sitzen, Stehen sieht eine vorteilhafte Ausführung vor, daß die Vorsteuereinrichtung einen Lagesensor, insbesondere Quecksilberschalter oder Wasserwaagen aufweist. Zur Erfassung von Bewegungszuständen ist es von Vorteil, wenn die Vorsteuereinrichtung einen Bewegungssensor, insbesondere einen Schrittzähler aufweist. Auch im Hinblick auf eine mittelbare Erfassung körperlicher Belastung ist es vorteilhaft, wenn die Vorsteuereinrichtung einen Fühler zur Erfassung von Körperparametern des Patienten wie Herzfrequenz, Körpertemperatur oder Hautleitfähigkeit aufweist.

Eine weitere bevorzugte Ausgestaltung der Erfindung sieht vor, daß die Vorsteuereinrichtung ein Basalsteuerglied zur kontinuierlichen Vorgabe einer vorzugsweise patientenbezogenen Basaldosis des Hormons aufweist. Damit läßt sich ein bestehender Grundbedarf decken, wobei der Regler in einem günstigen Arbeitspunkt bleibt. Eine weitere Verbesserung wird dadurch erreicht, daß das Basalsteuerglied eine Korrekturstufe zur Anpassung der Basaldosis an eine tageszeitveränderliche Hormonsensitivität des Patienten aufweist. Um auch die exogene Glukoseanflutung zu korrigieren, ist es von besonderem Vorteil, wenn die Vorsteuereinrichtung ein Bolussteuerglied zur Vorgabe von Hormonboli in Abhängigkeit der Nahrungsaufnahme des Patienten aufweist. Weiter ist es günstig, wenn die Vorsteuereinrichtung einen Zeitgeber zur zeitabhängigen Festlegung der Basaldosis oder zur Bestimmung des Zeitpunkts der Verabreichung von Hormonboli aufweist. Um zusätzlich den Einfluß körperlicher Aktivität in der Basal- und Bolusdosis berücksichtigen zu können, ist es vorteilhaft, wenn das Basalsteuerglied und/oder Bolussteuerglied der Aktivitätsmeßeinrichtung nachgeschaltet sind. Eine weitere vorteilhafte Ausgestaltung sieht vor, daß die Vorsteuereinrichtung eine Eingabeeinheit zur Dateneingabe insbesondere der Tageszeiten und Broteinheiten einer Nahrungsaufnahme des Patienten aufweist.

Die Einwirkung auf die Regeleinrichtung kann dadurch erfolgen, daß die Vorsteuereinrichtung einen mit dem Regler verbundenen Sollwertausgang zur Vorgabe einer Führungsgröße aufweist. Weitere vorteilhafte Möglichkeiten bestehen darin, daß die Vorsteuereinrichtung und der Regler ausgangsseitig über ein Summierglied mit der Hormondosiereinheit verbunden sind, oder daß die Vorsteuereinrichtung Mittel zur Auswahl des Regelalgorithmus oder zur Vorgabe von Reglerparametern des Reglers aufweist.

Um Fehlfunktionen zu verhindern, wird vorgeschlagen, daß die Vorsteuereinrichtung eine Überwachungsstufe zur Kontrolle und Begrenzung der Hormondosis gegebenenfalls nach Erfassung von kritischen Patientenzuständen wie abnorme Körpertemperatur aufweist.

Gemäß einer weiteren vorteilhaften Ausgestaltung der Erfindung weist die Meßeinrichtung einen vorzugsweise nach der Mikrodialysetechnik arbeitenden Glukosesensor zur Erfassung von Gewebeglukosewerten als Meßwerte auf. Damit ist eine fortlaufende Erfassung der Regelgröße möglich, ohne daß ein direkter Zugang zum Blutkreislauf aufrecht erhalten werden müßte. Hier kommt die Vorsteuerung besonders zur Geltung, weil der Glukoseübergang zwischen Blut und Gewebe mit einer gewissen Verzögerung erfolgt.

Soweit das zu applizierende Hormon durch Insulin gebildet ist, sollte der Stellbereich des Reglers auf positive Werte beschränkt sein. Grundsätzlich wäre es auch möglich, durch Dosierung von kontrainsulinären Hormonen wie Glukagon auch einen negativen Stellbereich zu nutzen. In jedem Fall ist es vorteilhaft, wenn die Vorsteuereinrichtung unter Einhaltung einer Stellreserve auf die Regeleinrichtung einwirkt.

Im Hinblick auf die Besonderheiten des Glukosestoffwechsels läßt sich ein optimales Reglerverhalten bzw. Regelergebnis dadurch erreichen, daß der Regler als Zustandsregler ausgebildet ist, wobei der Regelalgorithmus eine als Beobachter mit den Meß- und Hormondosiswerten beaufschlagbare Programmroutine zur Abschätzung nicht meßbarer Zustandsgrößen aufweist, und wobei der Regler ein Proportional-Integral-Glied zur Störgrößenbehandlung aufweist.

Als Stellglied der Regeleinrichtung wird vorgeschlagen, daß die Hormondosiereinheit durch eine zur vorzugsweise subcutanen Hormoninfusion ausgebildete Dosierpumpe gebildet ist. Aus Sicherheitsgründen kann der Regelkreis durch eine willentliche Handlung des Patienten geschlossen werden, vorteilhafterweise indem die Hormondosiereinheit ein Auslöseglied zur manuellen Bestätigung einer vorgesehenen Hormongabe aufweist. Grundsätzlich ist es im Hinblick auf eine Gefahrenreduzierung auch denkbar, daß die Hormondosiereinheit lediglich zum Anzeigen und/oder Bereitstellen bzw. Abmessen einer durch den Patienten selbst zu applizierenden Hormondosis ausgebildet ist.

Um eine flexible Lebensführung des Patienten zu ermöglichen, sieht eine vorteilhafte Ausgestaltung vor, daß die Meßeinrichtung, die Regeleinrichtung und die Vorsteuereinrichtung durch ein am Körper des Patienten tragbares Kleingerät gebildet sind.

In verfahrensmäßiger Hinsicht wird die vorstehend genannte Aufgabe dadurch gelöst, daß
- mit der Blutglukose korrelierbare Meßwerte erfaßt werden;
- die Meßwerte einer Regeleinrichtung zugeführt werden, wobei ein Regler die Meßwerte nach einem Regelalgorithmus verarbeitet und eine Hormondosiereinheit eine Hormondosis ausgibt;
- die zur Feindosierung des Hormons vorgesehene Regeleinrichtung (12) mittels einer Vorsteuereinrichtung (14) zur Totzeitverminderung vorgesteuert wird.

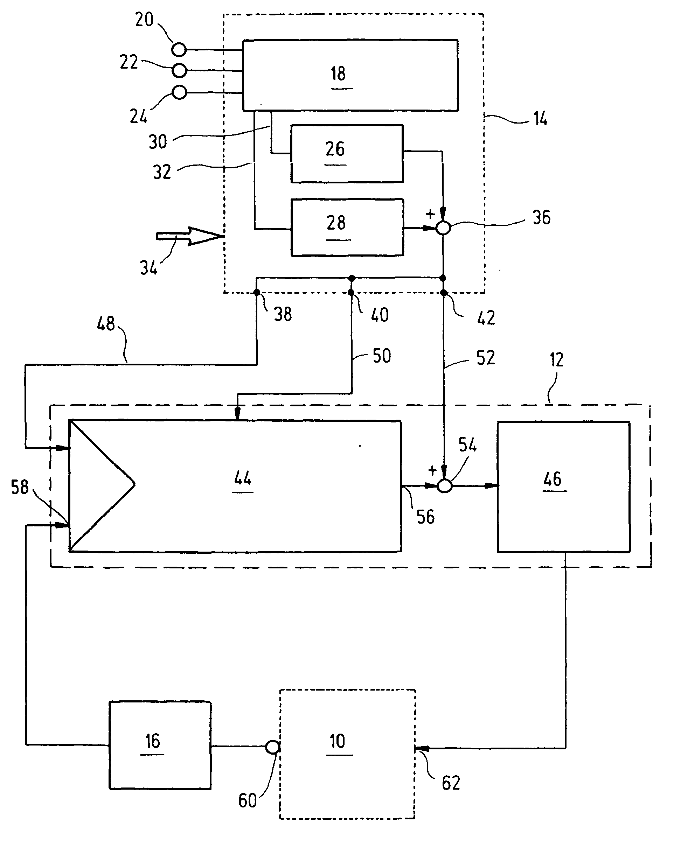

Im folgenden wird die Erfindung anhand eines in der Zeichnung in schematischer Weise dargestellten Ausführungsbeispiels näher erläutert. Die einzige Figur zeigt ein Blockschaltbild einer Anordnung bzw. eines Systems zur geregelten Insulindosierung.

Die in der Zeichnung dargestellte Anordnung ermöglicht die selbsttätige Regulierung der Blutglukose eines Diabetes-Patienten 10. Sie besteht im wesentlichen aus einer Regeleinrichtung 12 zur Feinausregelung der Insulinapplikation, einer Vorsteuereinrichtung 14 zur Grobvorsteuerung der Regeleinrichtung 12 nach Maßgabe mindestens einer den Blutglukosespiegel des Patienten 10 beeinflussenden Einfluß- bzw. Störgröße und einer Meßeinrichtung 16 zur sequentiellen Erfassung von mit dem Blutglukosespiegel korrelierten Meßwerten.

Die Vorsteuereinrichtung 14 weist eine Aktivitätsmeßeinheit 18 zur sensorischen Erfassung des körperlichen Aktivitätsgrades des Patienten als Einflußgröße auf. Zu diesem Zweck ist die Aktivitätsmeßeinheit 18 eingangsseitig mit einem zur Erfassung von Ruhezuständen des Patienten geeigneten Lagesensor 20 verbunden. Der Lagesensor 20 kann beispielsweise durch einen Quecksilberschalter oder gekreuzte Wasserwaagen gebildet sein, womit über die Neigungslage erfaßt werden kann, ob der Patient 10 liegt, sitzt oder steht. Daneben ist ein Bewegungssensor 22 dazu vorgesehen, der Aktivitätsmeßeinheit 18 geeignete Signale zur Erfassung von Bewegungszuständen des Patienten 10 zuzuführen. Hier kann ein Schrittzähler eingesetzt werden, um die körperliche Aktivität beim Gehen oder Laufen zu quantifizieren. Ein weiterer Fühler 24 dient zur Erfassung von Körperparametem des Patienten, welche zumindest mittelbar auf körperliche Aktivität schließen lassen, also insbesondere die Herzschlagrate, Körper- bzw. Hauttemperatur und Hautleitfähigkeit.

Die Vorsteuereinrichtung 14 umfaßt weiterhin ein Basalsteuerglied 26 zur Festlegung einer Basalrate bzw. Grunddosis, die geeignet ist, den Grundbedarf des Patienten an Insulin zu decken. Hierfür kann das Basalsteuerglied 26 eine nicht eigens gezeigte Korrekturstufe zur Anpassung der Basaldosis an eine zeitveränderliche periphere Insulinsensitivität aufweisen. Beispielsweise kann die Basaldosis entsprechend einer Rampenfunktion tageszeitabhängig verändert werden. Zum Ausgleich nahrungsbezogener exogener Glukoseanflutung als Einflußgröße weist die Vorsteuereinrichtung 14 ein Bolussteuerglied 28 auf, welches an die Nahrungsaufnahme angepaßte Insulinboli bzw. Insulingaben vorgibt. Dabei kann ein nicht gezeigter Zeitgeber bzw. eine Uhr zur Bestimmung des Verabreichungszeitpunkts der Insulingabe oder auch zur tageszeitabhängigen Zeitsteuerung der Basalrate vorgesehen sein. Die Steuerglieder 26, 28 sind über Signalpfade 30, 32 auf die Aktivitätsmeßeinheit 18 aufgeschaltet, so daß eine aktivitätsbezogene Gewichtung der Basal- und Boligaben möglich ist. Eine Eingabeeinheit 34 der Vorsteuereinrichtung 14 ermöglicht es, neben den sensorisch erfaßten Signalen zusätzliche Daten wie Tageszeiten und Broteinheiten einer Nahrungsaufnahme des Patienten zur Verarbeitung einzugeben. Ausgangsseitig werden die über ein Summierglied 36 zusammengeführten Steuersignale bzw. Ausgabedaten der Steuerglieder 26, 28 wahlweise einem der Ausgänge 38, 40, 42 der Vorsteuereinrichtung 14 zugeführt.

Zur korrigierenden Einwirkung auf die einen Regler 44 und eine Dosiereinheit 46 umfassende Regeleinrichtung 12 durch die Vorsteuereinrichtung 14 bestehen verschiedene Möglichkeiten. Über den Signalpfad 48 kann dem Regler 44 eine durch die Regelung nicht beeinflußbare Führungsgröße eingespeist werden, die den (zeitabhängigen) Sollwertverlauf festlegt. Die Datenstrecke 50 erlaubt es, einen geeigneten Regelalgorithmus auszuwählen oder Reglerparameter zu variieren. Zur unmittelbaren Einwirkung auf die Dosiereinheit 46 ist die Vorsteuereinrichtung 14 an dem Stellausgang 42 über die Leitung 52 und das Summierglied 54 additiv auf den Reglerausgang 56 aufschaltbar. Mit den genannten Maßnahmen ist es möglich, die Regeldifferenz auf einen kleineren Bereich zu begrenzen, während die Regeleinrichtung die Feindosierung übernimmt. Ergänzend ist es zweckmäßig, die auszugebende Insulindosis zu überwachen bzw. auf die Einhaltung von Plausibilitätskriterien zu kontrollieren. Dies wird durch eine nicht gezeigte Überwachungsstufe erreicht, die gegebenenfalls auch kritische Patientenzustände wie Krankheit beispielsweise anhand der Körpertemperatur erfaßt. Dabei sollte auch die Einhaltung einer Stell- bzw. Regelreserve durch entsprechende Begrenzung der Ausgangsgrößen der Vorsteuereinrichtung 14 gewährleistet sein.

Die Vorsteuereinrichtung 14 beeinflußt die Regeleinrichtung 12 in einem offenen Wirkungsablauf, d.h. die gesteuerte Ausgangsgröße (letztlich die Insulindosis) wirkt nicht auf die steuernde Einflußgröße (beispielsweise den Aktivitätsgrad des Patienten) zurück. Dabei erfolgt die Einflußnahme antizipierend bzw. vorsteuernd im Sinne einer sofortigen Korrektur der störenden Einwirkung, ohne erst eine Veränderung der Regelgröße abzuwarten. Hingegen arbeitet die Regelung prinzipiell in einem geschlossenen Kreis, in welchem eine Rückwirkung der anzugleichenden Größe auf die Meßgröße stattfindet.

Die Vorsteuereinrichtung 14 und der Regler 12 sind hardwaremäßig durch eine anwendungsspezifische integrierte Halbleiterschaltung oder ein geeignetes Prozessorsystem realisiert, insbesondere durch einen Mikrocontroller in Verbindung mit einem digitalen Signalprozessor. Darauf ist ein Zustandsregler hoher Regelgüte implementiert, bei welchem im Gegensatz zur herkömmlichen Ausgangsgrößenregelung die Zustandsgrößen der Regelstrecke rückgekoppelt werden. Zur Abschätzung von nicht unmittelbar meßbaren Zustandsgrößen weist der Regelalgorithmus eine als Beobachter mit den Insulinmeßwerten und den ausgegebenen Dosiswerten beaufschlagbare Programmroutine auf. Zusätzlich kommt ein Proportional-Integral-Glied zur Behandlung von Störeffekten wie exogene Glukoseanflutung zum Einsatz. Zur Synthese eines solchen Regelalgorithmus wird der Blutglukosestoffwechsel als mathematisches Modell durch ein lineares Differentialgleichungssystem erster Ordnung beschrieben, wobei Transport- bzw. Totzeiteffekte durch Proportional-Verzögerungs-(PT₁)-Glieder berücksichtigt werden. Grundsätzlich wäre es allerdings auch möglich, anstelle des vorstehend beschriebenen Zustandsreglers einen einfachen PID-Regler einzusetzen.

Zur Einspeisung von Ist- bzw. Meßwerten der Regelgröße ist der Regler 44 an seinem Eingang 58 mit der Meßeinrichtung 16 verbunden. Der Blutglukosespiegel wird wegen der Schwierigkeiten eines dauerhaften intravenösen Zugangs nicht unmittelbar erfaßt, sondern es wird der damit korrelierte Gewebeglukosespiegel im Unterhautfettgewebe des Patienten 10 gemessen. Zu diesem Zweck weist die Meßeinrichtung 16 einen Glukosesensor 60 auf, welcher in bekannter Weise nach der Mikrodialysetechnik arbeitet. Dabei wird eine im Gewebe implantierte Mikrodialysesonde mit einer Perfusionsflüssigkeit beaufschlagt und der Glukosegehalt durch einen nachgeordneten elektrochemisch-enzymatisch arbeitenden Elektrodensensor seuquentiell erfaßt. Die Meßwerte können dabei quasikontinuierlich oder aber intervallweise abgeleitet werden.

Die als Stellglied vorgesehene Dosiereinheit 46 ist durch eine Insulinpumpe gebildet, welche eine selbsttätige subcutane Insulinapplikation über eine Infusionskanüle 62 beispielsweise in der Bauchregion ermöglicht. Sowohl die Mikrodialysesonde als auch die Infusionskanüle können durch den Patienten ohne ärztliche Aufsicht selbst implantiert werden. Die aufgrund der Blut/Subcutanübergänge bei der Regelung auftretenden Totzeiteffekte lassen sich mit der vorgeschlagenen Regelstrategie problemlos bewältigen. Die gesamte Regel- und Steueranordnung läßt sich in einem am Körper des Patienten tragbaren Kleingerät unterbringen, welches damit die Funktion der Bauchspeicheldrüse zur normoglykämischen Stoffwechseleinstellung übernimmt.

## Patentansprüche

1. Anordnung zur Dosierung eines zur Regulierung der Blutglukose eines Patienten (10) geeigneten Hormons mit
a) einer Messeinrichtung (16) zur Erfassung von mit der Blutglukose korrelierbaren Messwerten;
b) einer Regeleinrichtung (12), die einen Regler (44) zur Verarbeitung der Messwerte nach einem Regelalgorithmus sowie eine Hormondosiereinheit (46) zur Ausgabe einer Hormondosis umfasst;
c) einer auf die Regeleinrichtung (12) einwirkenden Vorsteuereinrichtung (14) zur Grobvorsteuerung und Totzeitverminderung der Regelung nach Maßgabe einer den Blutglukosespiegel des Patienten beeinflussenden Einflussgröße
wobei
d) die Vorsteuereinrichtung (14) nach Maßgabe einer sensorisch erfassten Einflussgröße vorsteuernd auf die Regeleinrichtung (12) einwirkt.

2. Anordnung nach Anspruch 1, **dadurch gekennzeichnet, dass** die Vorsteuereinrichtung (14) eine Aktivitätsmesseinheit (18) zur sensorischen Erfassung des körperlichen Aktivitätsgrades des Patienten (10) aufweist

3. Anordnung nach Anspruch 1 oder 2, **dadurch gekennzeichnet, dass** die Vorsteuereinrichtung (14) einen Lagesensor (20), insbesondere Quecksllberschalter oder Wasserwaagen zur Erfassung von Ruhezuständen des Patienten (10) aufweist.

4. Anordnung nach einem der Ansprüche 1 bis 3, **dadurch gekennzeichnet, dass** die Vorsteuereinrichtung (14) einen Bewegungssensor (22), insbesondere Schrittzähler zur Erfassung von Bewegungszuständen des Patienten (10) aufweist.

5. Anordnung nach einem der Ansprüche 1 bis 4, **dadurch gekennzeichnet, dass** die Vorsteuereinnchtung (14) einen Fühler (24) zur Erfassung von Körperparametem des Patienten (10) wie Herzfrequenz, Körpertemperatur oder Hautleitfähigkeit aufweist.

6. Anordnung nach einem der Ansprüche 1 bis 5, **dadurch gekennzeichnet, dass** die Vorsteuereinrichtung (14) ein Basalsteuerglied (26) zur kontinuierlichen Vorgabe einer vorzugsweise patientenbezogenen **Basaldosis des Hormons aufweist.**

7. Anordnung nach Anspruch 6, **dadurch gekennzelchnet,** dass das Basalsteuerglied (26) eine Korrekturstufe zur Anpassung der Basaldosis an eine tageszeitveränderliche Hormonsensitivität des Patienten (10) aufweist.

8. Anordnung nach einem der Ansprüche 1 bis 7, **dadurch gekennzeichnet, dass** die Vorsteuereinrichtung (14) ein Bolussteuerglied (28) zur Vorgabe von Hormonboli in Abhängigkeit der Nahrungsaufnahme des Patienten (10) aufweist.

9. Anordnung nach einem der Ansprüche 1 bis 8, **dadurch gekennzeichnet, dass** die Vorsteuereinrichtung (14) einen Zeitgeber zur zeitabhängigen Festlegung einer Hormonbasaldosis oder zur Bestimmung des Zeitpunkts der Verabreichung von Hormonboli aufweist.

10. Anordnung nach einem der Ansprüche 6 bis 9, **dadurch gekennzeichnet, dass** das Basalsteuerglied (26) und/oder Bolussteuerglied (28) der Aktivitätsmesseinheit (18) nachgeschaltet sind.

11. Anordnung nach einem der Ansprüche 1 bis 10, **dadurch gekennzeichnet, dass** die Vorsteuereinrichtung (14) eine Eingabeeinheit (34) zur Dateneingabe insbesondere der Tageszeiten und Broteinheiten einer Nahrungsaufnahme des Patienten (10) aufweist.

12. Anordnung nach einem der Ansprüche 1 bis 11, **dadurch gekennzeichnet, dass** die Vorsteuereinrichtung (14) einen mit dem Regler (44) verbundenen Sollwertausgang (38) zur Vorgabe einer Führungsgröße aufweist

13. Anordnung nach einem der Ansprüche 1 bis 12, **dadurch gekennzeichnet, dass** die Vorsteuereinrichtung (14) und der Regler (44) ausgangsseitig über ein Summierglied (54) mit der Hormondosiereinheit (46) verbunden sind.

14. Anordnung nach einem der Ansprüche 1 bis 13, **dadurch gekennzeichnet, dass** die Vorsteuereinrichtung (14) Mittel (40,50) zur Auswahl des Regelalgorithmus oder zur Vorgabe von Parametem des Reglers (44) aufweist.

15. Anordnung nach einem der Ansprüche 1 bis 14, **dadurch gekennzeichnet, dass** die Vorsteuereinrichtung (14) eine Überwachungsstufe zur Kontrolle und Begrenzung der Hormondosis gegebenenfalls nach Erfassung von kritischen Patientenzuständen wie abnorme Körpertemperatur aufweist.

16. Anordnung nach einem der Ansprüche 1 bis 15. **dadurch gekennzeichnet, dass** die Messeinrichtung (16) einen vorzugsweise nach der Mikrvdialysetechnik arbeitenden Glukosesensor (60) zur Erfassung von Gewebeglukosewerten als Messwerte aufweist.

17. Anordnung nach einem der Ansprüche 1 bis 16, **dadurch gekennzeichnet, dass** der Stellbereich des Reglers (44) auf positive Werte beschränkt ist, wobei das Hormon durch Insulin gebildet ist.

18. Anordnung nach einem der Ansprüche 1 bis 17, **dadurch gekennzeichnet, dass** die Vorsteuereinrichtung (14) unter Einhaltung einer Stellreserve auf die Regeleinrichtung (12) einwirkt.

19. Anordnung nach einem der Ansprüche 1 bis 18, **dadurch gekennzeichnet, dass** der Regler (44) als Zustandsregler ausgebildet ist, wobei der Regelalgorithmus eine als Beobachter mit den Mess- und Hormondosiswerten beaufschlagbare Programmroutine zur Abschätzung nicht messbarer Zustandsgrößen aufweist.

20. Anordnung nach einem der Ansprüche 1 bis 19, **dadurch gekennzeichnet, dass** der Regler (44) ein Proportional-Integral-Glied zur Störgrößenbehandlung aufweist.

21. Anordnung nach einem der Ansprüche 1 bis 20, **dadurch gekennzeichnet, dass** die Hormondosiereinheit (46) als Stellglied der Regeleinrichtung (12) durch eine zur vorzugsweise subcutanen Hormoninfusion ausgebildete Dosierpumpe (46) gebildet ist

22. Anordnung nach einem der Ansprüche 1 bis 21, **dadurch gekennzeichnet, dass** die Hormondosiereinheit (46) ein Auslöseglied zur manuellen Bestätigung einer vorgesehenen Hormongabe aufweist.

23. Anordnung nach einem der Ansprüche 1 bis 22, **dadurch gekennzeichnet, dass** die Hormondosiereinheit (46) zum Anzeigen und/oder Bereitstellen einer durch den Patienten zu applizierenden Hormondosis ausgebildet ist.

24. Anordnung nach einem der Ansprüche 1 bis 23, **dadurch gekennzeichnet, dass** die Messeinrichtung (16), die Regeleinrichtung (12) und die Vorsteuereinrichtung (14) durch ein am Körper des Patienten (10) tragbares Kleingerät gebildet sind.

## Claims

1. Array for dosing a hormone suitable for regulating the blood glucose of a patient (10) comprising
a) a measuring device (16) for detecting measured values correlatable with blood glucose;
b) controlling means (12) comprising a controller (44) to process the measured values according to a control algorithm and a hormone dosing unit (46) to administer a hormone dose;
c) a pilot control device (14) acting on the controlling means (12) to perform coarse pre-control and reduce dead time of the control in accordance with an influencing variable which influences the blood glucose level of the patient;
wherein
d) the pilot control device (14) has a pre-controlling action on the controlling means (12) in accordance with at least one influencing variable that is detected by a sensor.

2. Array as claimed in claim 1, **characterized in that** the pilot control device (14) has an activity measuring unit (18) for the sensory detection of the degree of physical activity of the patient (10).

3. Array as claimed in claim 1 or 2, **characterized in that** the pilot control device (14) has a position sensor (20) and in particular mercury switches or spirit levels to detect resting states of the patient (10).

4. Array as claimed in one of the claims 1 to 3, **characterized in that** the pilot control device (14) has a movement sensor (22) and in particular a pedometer to detect movement states of the patient (10).

5. Array as claimed in one of the claims 1 to 4, **characterized in that** the pilot control device (14) has a sensor (24) to detect body parameters of the patient (10) such as heart-rate, body temperature or skin conductivity.

6. Array as claimed in one of the claims 1 to 5, **characterized in that** the pilot control device (14) has a basal control element (26) for the continuous pre-administration of a preferably patient-related basal dose of the hormone.

7. Array as claimed in claim 6, **characterized in that** the basal control element (26) has a correction stage to adjust the basal dose to the diurnal variations of the hormone sensitivity of the patient (10).

8. Array as claimed in one of the claims 1 to 7, **characterized in that** the pilot control device (14) has a bolus control element (28) to pre-administer hormone boli depending on the food intake of the patient (10).

9. Array as claimed in one of the claims 1 to 8, **characterized in that** the pilot control device (14) has a timer for the time-dependent determination of a basal hormone dose or to determine the time for the administration of hormone boli.

10. Array as claimed in one of the claims 6 to 9, **characterized in that** the basal control element (26) and/or the bolus control element (28) are connected downstream of the activity measuring unit (18).

11. Array as claimed in one of the claims 1 to 10, **characterized in that** the pilot control device (14) has an input unit (34) to enter data and in particular the times of day and bread units of the patient's food intake (10).

12. Array as claimed in one of the claims 1 to 11, **characterized in that** the pilot control device (14) has a set-point output (38) connected to the controller (44) in order to pre-set a command variable.

13. Array as claimed in one of the claims 1 to 12, **characterized in that** the pilot control device (14) and the controller (44) are connected on the output side via a summation element (54) to the hormone dosage unit (46).

14. Array as claimed in one of the claims 1 to 13, **characterized in that** the pilot control device (14) has means (40, 50) for selecting the control algorithm or to pre-set parameters of the controller (44).

15. Array as claimed in one of the claims 1 to 14, **characterized in that** the pilot control device (14) has a monitoring stage to monitor and limit the hormone dose optionally after registering critical conditions of the patient such as abnormal body temperature.

16. Array as claimed in one of the claims 1 to 15, **characterized in that** the measuring device (16) has a glucose sensor (60) which preferably utilizes microdialysis technology to detect tissue glucose values as measured values.

17. Array as claimed in one of the claims 1 to 16, **characterized in that** the operating range of the controller (44) is limited to positive values when the hormone is insulin.

18. Array as claimed in one of the claims 1 to 17, **characterized in that** the pilot control device (14) acts on the controlling means (12) while maintaining a reserve for adjustment.

19. Array as claimed in one of the claims 1 to 18, **characterized in that** the controller (44) is designed as a state controller in which the control algorithm has a program routine acting as observer of the measured values and hormone dosage values in order to estimate non-measurable variables of state.

20. Array as claimed in one of the claims 1 to 19, **characterized in that** the controller (44) has a proportional-integral element to deal with disturbance variables.

21. Array as claimed in one of the claims 1 to 20, **characterized in that** the hormone dosage unit (46) comprises a dosage pump (46) designed for preferably subcutaneous hormone infusion as the actuator of the controlling means (12).

22. Array as claimed in one of the claims 1 to 21, **characterized in that** the hormone dosage unit (46) has a triggering element to manually confirm a planned hormone administration.

23. Array as claimed in one of the claims 1 to 22, **characterized in that** the hormone dosage unit (46) is designed to display and/or provide a hormone dose which is administered by the patient himself.

24. Array as claimed in one of the claims 1 to 23, **characterized in that** the measuring device (16), the controlling means (12) and the pilot control device (14) are in the form of a small portable instrument that can be carried on the body of the patient (10).

## Revendications

1. Système pour le dosage d'une hormone adéquate pour réguler le glucose sanguin d'un patient (10), avec
a) un dispositif de mesure (16) pour acquérir des valeurs de mesure corrélables avec le glucose sanguin ;
b) un dispositif régulateur (12) qui comprend un régulateur (44) pour le traitement des valeurs de mesure suivant un algorithme de régulation ainsi qu'un module de dosage hormonal (46) pour délivrer une dose d'hormone ;
c) un dispositif de prérégulation (14) agissant sur le dispositif régulateur (12) pour une prérégulation grossière et une réduction des temps morts de la régulation en fonction d'une grandeur d'influence qui influe sur le taux de glucose sanguin du patient
dans lequel
d) le dispositif de prérégulation (14) agit en pilotant le dispositif régulateur (12) en fonction d'une grandeur d'influence détectée par un capteur.

2. Système selon la revendication 1, **caractérisé en ce que** le dispositif de prérégulation (14) présente un module de mesure de l'activité (18) afin de détecter au moyen de capteurs le degré d'activité physique du patient (10).

3. Système selon la revendication 1 ou la revendication 2, **caractérisé en ce que** le dispositif de prérégulation (14) présente un capteur de position (20), en particulier un interrupteur au mercure ou des niveaux à bulle pour détecter des états de repos du patient (10) .

4. Système selon l'une des revendications 1 à 3, **caractérisé en ce que** le dispositif de prérégulation (14) présente un capteur de déplacement (22), en particulier un podomètre, pour détecter des états en mouvement du patient (10).

5. Système selon l'une des revendications 1 à 4, **caractérisé en ce que** le dispositif de prérégulation (14) présente une sonde (24) pour détecter des paramètres corporels du patient (10) tels que la fréquence cardiaque, la température corporelle ou la conductivité cutanée.

6. Système selon l'une des revendications 1 à 5, **caractérisé en ce que** le dispositif de prérégulation (14) présente un élément de réglage basal (26) pour prédéterminer en continu une dose basale de l'hormone, de préférence spécifique au patient.

7. Système selon la revendication 6, **caractérisé en ce que** l'élément de réglage basal (26) présente un étage de correction pour adapter la dose basale à une sensibilité hormonale du patient (10) qui varie en fonction du moment de la journée.

8. Système selon l'une des revendications 1 à 7, **caractérisé en ce que** le dispositif de prérégulation (14) présente un élément de réglage de bolus (28) pour prédéterminer des bolus hormonaux en fonction de la prise d'aliment du patient (10).

9. Système selon l'une des revendications 1 à 8, **caractérisé en ce que** le dispositif de prérégulation (14) présente un générateur de base de temps pour déterminer une dose basale d'hormone en fonction du temps ou pour déterminer le moment de l'administration de bolus hormonaux.

10. Système selon l'une des revendications 6 à 9, **caractérisé en ce que** l'élément de réglage basal (26) et/ou l'élément de réglage de bolus (28) sont montés en aval du module de mesure de l'activité (18).

11. Système selon l'une des revendications 1 à 10, **caractérisé en ce que** le dispositif de prérégulation (14) présente un module de saisie (34) pour la saisie de données, en particulier des heures de la journée et des unités de pain d'une prise d'aliment du patient (10).

12. Système selon l'une des revendications 1 à 11, **caractérisé en ce que** le dispositif de prérégulation (14) présente une sortie de valeur de consigne (38) reliée au régulateur (44) pour prédéfinir une grandeur de référence.

13. Système selon l'une des revendications 1 à 12, **caractérisé en ce que** le dispositif de prérégulation (14) et le régulateur (44) sont reliés à leur sortie par le biais d'un élément sommateur (54) au module de dosage hormonal (46).

14. Système selon l'une des revendications 1 à 13, **caractérisé en ce que** le dispositif de prérégulation (14) présente des moyens (40, 50) pour sélectionner l'algorithme de régulation ou pour prédéfinir des paramètres du régulateur (44).

15. Système selon l'une des revendications 1 à 14, **caractérisé en ce que** le dispositif de prérégulation (14) présente un étage de surveillance pour contrôler et limiter la dose d'hormone, le cas échéant après la détection d'états critiques du patient tels qu'une température corporelle anormale.

16. Système selon l'une des revendications 1 à 15, **caractérisé en ce que** le dispositif de mesure (16) présente un capteur de glucose (60), fonctionnant de préférence suivant la technique de la microdialyse, pour détecter des taux de glucose tissulaire en tant que valeurs de mesure.

17. Système selon l'une des revendications 1 à 16, **caractérisé en ce que** la plage de réglage du régulateur (44) est limitée à des valeurs positives, l'hormone étant constituée par de l'insuline.

18. Système selon l'une des revendications 1 à 17, **caractérisé en ce que** le dispositif de prérégulation (14) agit sur le dispositif régulateur (12) en conservant une réserve de réglage.

19. Système selon l'une des revendications 1 à 18, **caractérisé en ce que** le régulateur (44) est réalisé sous la forme d'un régulateur d'état, l'algorithme de régulation présentant une routine logicielle en tant que module de surveillance auquel les valeurs de mesure et de dose d'hormone peuvent être appliquées afin d'estimer une grandeur d'état non mesurable.

20. Système selon l'une des revendications 1 à 19, **caractérisé en ce que** le régulateur (44) présente un élément proportionnel et intégral pour le traitement des grandeurs perturbatrices.

21. Système selon l'une des revendications 1 à 20, **caractérisé en ce que** le module de dosage hormonal (46) est réalisé en tant qu'organe de réglage du dispositif régulateur (12) par une pompe doseuse (46) conçue de préférence pour une perfusion hormonale sous-cutanée.

22. Système selon l'une des revendications 1 à 21, **caractérisé en ce que** le module de dosage hormonal (46) présente un élément déclencheur pour activer manuellement une administration d'hormone prévue.

23. Système selon l'une des revendications 1 à 22, **caractérisé en ce que** le module de dosage hormonal (46) est réalisé de manière à afficher et/ou préparer une dose d'hormone que le patient doit s'administrer.

24. Système selon l'une des revendications 1 à 23, **caractérisé en ce que** le dispositif de mesure (16), le dispositif régulateur (12) et le dispositif de pré- régulation (14) sont formés par un appareil miniature que le patient (10) peut porter sur le corps.
